Europäisches Patentamt

European Patent Office

Office européen des brevets

(19)

(11) Veröffentlichungsnummer : **0 160 162 B1**

# (12) EUROPÄISCHE PATENTSCHRIFT

(45) Veröffentlichungstag der Patentschrift :
29.07.87

(21) Anmeldenummer : 85100641.1

(22) Anmeldetag : 23.01.85

(51) Int. Cl.⁴ : **C 07 C 97/26**

(54) Verfahren zur Herstellung von 1-Amino-2-brom-4-hydroxyanthrachinon.

(30) Priorität : 28.04.84 DE 3415917

(43) Veröffentlichungstag der Anmeldung :
06.11.85 Patentblatt 85/45

(45) Bekanntmachung des Hinweises auf die Patenterteilung : 29.07.87 Patentblatt 87/31

(84) Benannte Vertragsstaaten :
CH DE FR GB LI

(56) Entgegenhaltungen :
DE-A- 2 817 890
DE-B- 2 713 575
JP-A-12 924 980
PATENT ABSTRACTS OF JAPAN, unexamined applications, Sektion C, Band 3, Nr. 11, 30. Januar 1979,
THE PATENT OFFICE JAPANESE GOVERNMENT,
Seite 132 C 35

(73) Patentinhaber : BAYER AG
Konzernverwaltung RP Patentabteilung
D-5090 Leverkusen 1 Bayerwerk (DE)

(72) Erfinder : Dittmer, Helmut, Dr.
Zedernweg 6
D-5090 Leverkusen 3 (DE)
Erfinder : Schneider, Jürgen, Dr.
Am Thelensiefen 9
D-5068 Odenthal (DE)
Erfinder : Schönhagen, Hubert
Im Schwarzbroich 15
D-5068 Odenthal (DE)
Erfinder : Krügermann, Claus
Lützenkirchener Strasse 143
D-5090 Leverkusen 3 (DE)

## Beschreibung

Gegenstand der Erfindung ist ein Verfahren zur Herstellung von 1-Amino-2-brom-4-hydroxyanthrachinon durch Bromierung von 1-Aminoanthrachinon in konzentrierter Schwefelsäure und anschließende Hydroxylierung in Gegenwart von Borsäure, wobei das Bromierungsprodukt nicht zwischenisoliert wird.

Derartige « Eintopfverfahren » sind bereits mehrfach in der Literatur beschrieben wurden (vgl. z. B. DE-PS 2 713 575 und 2 817 890). Dabei wird in der Regel die Brommenge so bemessen, daß möglichst quantitativ das 1-Amino-2,4-dibromanthrachinon entsteht, welches dann nach Zugabe von Borsäure ohne Zwischenisolierung hydrolysiert wird.

Diese Herstellungsmethoden, welche zweifelsohne gegenüber den bislang üblichen Zweistufenverfahren eine bedeutende Verfahrenstechnische Vereinfachung darstellen, weisen indessen den Nachteil auf, daß sie mit verhältnismäßig großen Mengen an Schwefelsäure und Brom arbeiten, was nicht nur die Herstellungskosten erheblich belastet, sondern auch die Abwasseraufbereitung erschwert.

Es wurde nun gefunden, daß man diese Nachteile in ihrer Auswirkung deutlich reduzieren kann, wenn man die Bromierung in der 2- bis 5-fachen Gewichtsmenge 80- bis 98 %-iger Schwefelsäure (« Startkonzentration ») und mit 1,1-1,5 Mol Brom — jeweils bezogen auf 1-Aminoanthrachinon — bei erhöhtem Druck durchgeführt.

Ansonsten erfolgt sowohl die Bromierung als auch die Hydrolyse unter üblichen Bedingungen.

Dabei werden praktisch die gleichen Ausbeuten und Reinheiten erzielt wie bei den wesentlich verdünnter bzw. mit höheren Brommengen arbeitenden Verfahren des Standes der Technik, was als ausgesprochen überraschend angesehen werden muß, weil normalerweise in konzentrierten Medien Produkte mit geringeren Reinheitsgraden anfallen.

Bei den erfindungsgemäßen Einsatz von unteräquimolaren Mengen Brom wird bewußt in Kauf genommen, daß das Bromierungsprodukt zum Zeitpunkt des Hydrolysenstarts 30-40 % an 1-Amino-2-brom-anthrachinon (neben dem 2,4-Dibromderivat) enthält.

Die Tatsache, daß trotzdem ein qualitativ hochwertiges Hydrolyseprodukt entsteht, muß ebenfalls als überraschend bezeichnet werden, da in den obengenannten Patentschriften die Lehre erteilt wird, daß ein großer Anteil an jenem Monobromderivat die Reinheit des Hydrolyseprodukts nachteilig beeinflußt.

Vorzugsweise wird der Bromierungsschritt in der 2,5-4-fachen Menge an 90-96 %iger Schwefelsäure bei 60-120 °C, insbesondere bei 70-100 °C, unter einem Überdruck von 0,5-5 bar, insbesondere 1-4 bar, in einem Autoklaven durchgeführt. Das Ende der Bromierung ist dann erreicht, wenn in einer entnommenen Probe praktisch kein 1-Aminoanthrachinon mehr nachweisbar ist.

Bevorzugt werden 1,2-1,4 Mol Brom eingesetzt.

Die Verseifung des Bromierungsgemisches erfolgt in Gegenwart von Borsäure (ca. 2-3 Mol bezogen auf 1-Aminoanthrachinon), wobei durch Zugabe von Oleum eine Schwefelsäurekonzentration von 95-110 % eingestellt wird. Die Temperaturen liegen bei 100-130 °C.

All diese Bedingungen sowie die Aufarbeitung durch Austragen in Wasser gehören zum Stand der Technik.

Eine besondere erfindungsgemäße Variante besteht indessen darin, daß man die Borsäure von Anfang an zusetzt. Auf diese Weise wird das neue Eintopfverfahren noch weiter vereinfacht.

Man trägt dann bei der praktischen Durchführung des Verfahrens in die vorgelegte Schwefelsäure 1-Aminoanthrachinon, Borsäure und Brom bei Raumtemperatur ein, schließt das Druckgefäß und erhitzt auf die gewünschte Reaktionstemperatur. Nach der Bromierung wird der Autoklav entspannt und mit der benötigten Oleummenge beschickt. Dann wird ohne weiteren technischen Aufwand die Reaktion bei üblichen Temperaturen zu Ende geführt.

Beispiel 1

In einem Druckkessel werden zu einer Mischung von 10,0 kg 1-Aminoanthrachinon (97 %) und 15 l 98 %iger Schwefelsäure bei 50 °C 9,0 kg Brom gegeben. Dann wird das Reaktionsgefäß fest verschlossen und der Gefäßinhalt 1 Stunde bei 70 °C 1 Stunde bei 80 °C und schließlich 2 Stunden bei 90 °C gut gerührt.

Danach wird auf 50 °C abgekühlt und der Kessel langsam entspannt.

Das Bromierungsgemisch wird ohne Zwischenisolierung nacheinander vorsichtig mit 5,5 kg Borsäure und 27 l Oleum (20 %) versetzt, wobei unter Kühlung die Temperatur bei 50°-60 °C gehalten wird, ehe man auf 75 °C erhitzt (1 Stunde) und weiter jeweils 1 Stunde lang auf 90 °C und 100 °C erhöht. Schließlich wird auf 115 °C erhitzt und 6 Stunden bei dieser Temperatur nachgerührt, wobei das entstehende Brom abdestilliert.

Nach dem Abkühlen auf 30-40 °C wird überschüssiges Brom im Vakuum abgezogen und das Reaktionsgemisch auf 80 l Wasser ausgetragen. Dabei steigt die Temperatur bis auf 90 °C an. Durch Einblasen von Dampf wird die Temperatur auf 95 °C erhöht. Man läßt noch 2 Stunden bei dieser Temperatur rühren und saugt dann das Produkt auf einer Nutsche ab. Nach dem Waschen mit heißem Wasser und Trocknen erhält man — umgerechnet auf 100 %ige Ware — 12,4 kg 1-Amino-2-brom-4-hydroxyanthrachinon was einer Ausbeute von 89,5 % entspricht das Produkt enthält weniger als 0,5 % 1-Amino-4-hydroxyanthrachinon, je ca. 1 % 1-Amino-2,4-dihydroxyanthrachinon und 1-Amino-2,4-dibromanthrachinon sowie Spuren an anderen Bromderivaten.

Beispiel 2

In einem Druckkessel werden je 12 l Schwefelsäure (96 %) und Oleum (20 %) vorgelegt. Unter Rühren werden 8,25 kg Borsäure eingetragen, wobei die Temperatur bis 60 °C ansteigen kann. Nachdem sich die Borsäure vollständig gelöst hat wird die Lösung bei 40-50 °C mit 15 kg 1-Aminoanthrachinon versetzt. Man läßt 1 Stunde nachrühren und danach vorsichtig 13,5 kg Brom zulaufen.

Der Autoklav wird nun dicht verschlossen und 1 Stunde bei 70 °C, 1 Stunde bei 80 °C und 2 Stunden bei 90 °C gerührt, wobei sich ein Druck von 3,4-4 bar einstellt. Nach dem Abkühlen und Entspannen läßt man langsam bei 50-65 °C 34,5 l Oleum (20 %) zufließen. Dann erhitzt man allmählich — wie in Beispiel 1 angegeben — auf 115 °C und hält bei dieser Temperatur 6 Stunden. Danach Aufarbeitung wie in Beispiel 1.

Man erhält schließlich 18,8 kg 1-Amino-2-brom-4-hydroxyanthrachinon (umgerechnet auf 100 %ige Ware), was einer Ausbeute von 90,4 % der Theorie entspricht.

Beispiel 3

Ein Rührautoklav wird nacheinander mit 40 kg Schwefelsäure (80 %ig), 10 kg 1-Aminoanthrachinon (97 %ig) und 3,4 l Brom beschickt.

Nach dem Verschließen des Druckgefäßes wird stufenweise auf 90 °C erhitzt und 2 Stunden bei dieser Temperatur gehalten. Man läßt auf Raumtemperatur abkühlen und entspannt das Gefäß.

Dann werden 5.5 kg Borsäure eingetragen und gut verrührt. Anschließend läßt man vorsichtig unter guter Kühlung 66 kg Oleum (65 %ig) zulaufen. Die Innentemperatur steigt dabei auf 70 °C an. Dann wird stufenweise auf 115 °C erhitzt und 6 Stunden bei dieser Temperatur gerührt. Danach wird die Reaktionsschmelze auf 80 l Eiswasser ausgetragen. Das dabei erhaltene Gemisch wird 2 Stunden bei 95 °C gerührt und anschließend auf eine Filterpresse gegeben. Nach dem Neutralwaschen und Trocknen erhält man — umgerechnet auf 100 %ige Ware — 12.06 kg (87 % der Theorie) eines Produkts, das aus 91.4 % 1-Amino-2-brom-4-hydroxyanthrachinon und 2.3 % 1-Amino-2,4-dibromanthrachinon besteht. Der Rest wurde analytisch nicht identifiziert.

Beispiel 4

Ein ähnlich gutes Ergebnis wie in Beispiel 3 wird erzielt, wenn man statt 3.4 l Brom nur 2.7 l Brom einsetzt.

## Patentansprüche

1. Verfahren zur Herstellung von 1-Amino-2-brom-4-hydroxyanthrachinon durch Bromierung von 1-Aminoanthrachinon in konzentrierter Schwefelsäure und Hydroxylierung des Bromierungsprodukts ohne Zwischenisolierung in Gegenwart von Borsäure unter sonst üblichen Bedingungen dadurch gekennzeichnet, daß man die Bromierung in der 2- bis 5-fachen Gewichtsmenge an 80- bis 98 %iger Schwefelsäure und mit 1,1-1,5 Mol Brom — jeweils bezogen auf 1-Aminoanthrachinon — bei erhöhtem Druck durchführt.

2. Verfahren nach Anspruch 1, dadurch gekennzeichnet, daß man in der 2,5- bis 4-fachen Menge an Schwefelsäure arbeitet.

3. Verfahren nach Anspruch 1, dadurch gekennzeichnet, daß man 1,2-1,4 Mol Brom einsetzt.

4. Verfahren nach Anspruch 1, dadurch gekennzeichnet, daß man mit einem Überdruck von 1-5 bar arbeitet.

5. Verfahren nach Anspruch 1, dadurch gekennzeichnet, daß man die Borsäure von Anfang an zusetzt.

## Claims

1. Process for the preparation of 1-amino-2-bromo-4-hydroxyanthraquinone by bromination of 1-aminoanthraquinone in concentrated sulphuric acid and hydroxylation of the bromination product without intermediate isolation in the presence of boric acid under conditions which are otherwise customary, characterised in that the bromination is carried out in 2 to 5 times the quantity by weight of 80 to 98 % sulphuric acid and using 1.1-1.5 mole of bromine — in each case relative to 1-aminoanthraquinone — under elevated pressure.

2. Process according to Claim 1, characterised in that it is carried out in 2.5 to 4 times the quantity of sulphuric acid.

3. Process according to Claim 1, characterised in that 1.2-1.4 mole of bromine is used.

4. Process according to Claim 1, characterised in that it is carried out under an excess pressure of 1-5 bar.

5. Process according to Claim 1, characterised in that the boric acid is added at the start.

## Revendications

1. Procédé pour la fabrication de 1-amino-2-bromo-4-hydroxyanthraquinone par bromation de 1-aminoanthraquinone dans l'acide sulfurique concentré et hydroxylation du produit de bromation sans isolement intermédiaire en présence d'acide borique dans des conditions par ailleurs habituelles, caractérisé en ce que l'on met en œuvre la bromation de la 1-aminoanthraquinone sous pression élevée dans 2 à 5 fois son poids d'acide sulfurique à 80-98 % et avec 1,1-1,5 mole de brome, par mole de 1-aminoanthraquinone.

2. Procédé selon la revendication 1, caractérisé en ce que l'on opère dans 2,5 à 4 fois la quantité en acide sulfurique.

3. Procédé selon la revendication 1, caractérisé en ce que l'on utilise 1,2-1,4 mole de brome.

4. Procédé selon la revendication 1, caractérisé en ce que l'on opère avec une surpression de 1-5 bars.

5. Procédé selon la revendication 1, caractérisé en ce que l'on ajoute l'acide borique dès le début.